# EUROPEAN PATENT APPLICATION

(11) **EP 4 273 251 A1**
(43) Date of publication of application: **08.11.2023**
(21) Application number: 21915742.7
(22) Date of filing: 28.12.2021
(51) Int. Cl.: C12N 15/75, C12N 9/10, C12P 19/18

(54) **RECOMBINANT BACILLUS SP. MICROORGANISM AND METHOD FOR PRODUCING HUMAN MILK OLIGOSACCHARIDES USING SAME**

(30) Priority: 31.12.2020 KR 20200189491
(71) Applicant: Samyang Corporation, Jongno-gu Seoul 03129 (KR)
(72) Inventor: HONG, Eun-Young, Seoul 07340 (KR); PARK, Bu-Soo, Hanam-si, Gyeonggi-do 13014 (KR); LEE, Sanghee, Gwangju-si, Gyeonggi-do 12788 (KR); KWON, Soun Gyu, Gwangmyeong-si, Gyeonggi-do 14311 (KR); CHOI, Eunsoo, Seongnam-si, Gyeonggi-do 13588 (KR)
(74) Representative: Schrell, Andreas
(86) International application number: PCT/KR2021/020000
(87) International publication number: WO 2022/145944

(57) **Abstract**

The present invention relates to a recombinant *Bacillus* sp. microorganism and a method for producing human milk oligosaccharides using the same.

## Description

### [TECHNICAL FIELD]

The present invention relates to a recombinant *Bacillus* sp. microorganism producing fucosyl transferase and a method for producing human milk oligosaccharides using the same.

### [BACKGROUND ART]

137 kinds including 3 kinds of oligosaccharides with the highest contents among human milk are fucosylated and ratio thereof is about 77%, and the rest oligosaccharides are mostly sialylated (39 kinds) and correspond to about 28%. Among them, in particular, 2'-fucosyllactose and 3'-fucosyllactose have a prebiotic effect, an effect of inhibiting intestinal attachment of pathogens, and an effect of regulating an immunoregulatory system, and the like. Prebiotic effect has been the most researched so far, and human milk oligosaccharides of breast milk selectively enhance growth and development of *Bifidobacterium bifidum,* which grows as a dominant species initially in intestines of infants, whereas they cannot be used by harmful bacteria. In addition, the human milk oligosaccharides are known to inhibit intestinal attachment of pathogens, and this is because structures of cell wall polysaccharides of a pathogen that binds to intestinal lectin is often similar to some structures of human milk oligosaccharides. Therefore, it seems that breast-feeding infants have resistance to infection by pathogens as human milk oligosaccharides provide watersoluble ligand analogues. However, it is known that due to mutation of fucosyl transferase which synthesizes 2'-fucosyllactose, about 20% of women cannot synthesize it in the body properly. Due to this, industrial production of 2'-fucosyllactose is required.

Some of human milk oligosaccharides are found also in milk of most mammals (primates, cattle, pigs, goats, sheep, elephants) in a small amount. Among them, the milk of goats is most similar to the composition of the human milk oligosaccharides, and due to this, a large-scale membrane use separation technology from whey by-products of a goat milk cheese production process has been suggested. In addition, when recently developed solid-phase synthesis is used, Lewis X-Lewis Y saccharides can be produced within one day, and when a crystalline intermediate technology is used, rapid synthesis of 2'-fucosyllactose becomes possible. However, despite development of the above separation method or a chemical synthesis method, several obstacles remain for mass production for industrialization of human milk oligosaccharides, as the present technologies are not suitable for a food or pharmaceutical industry due to low stereoselectivity, low total yield and use of toxic reagents, and the like. Thus, more environmentally friendly and high yield producing method is required, and recently, a method for producing a highconcentration 2'-FL by a microorganism has been reported.

However, a 2'-fucosyllactose synthesis gene uses 2-fucosyllactose secured from a Bio Safety Level 2 strain, *Helicobacter pylori*, and thus, it may act as a negative factor in perception of consumers when applied mainly to powdered milk for replacing breast-feeding and food for infants, and it is not appropriate for domestic production and sales permission. Therefore, it is needed to produce 2'-fucosyllactose as a food additive through development of a Bio Safety Level 1 strain by applying a gene with higher stability.

### [DISCLOSURE]

### [TECHNICAL PROBLEM]

An embodiment of the present invention is to provide a gene expression cassette expressing α-1,2-fucosyl transferase in a *Bacillus* sp. microorganism, comprising a nucleic acid sequence encoding α-1,2-fucosyl transferase, and a regulatory sequence operably linked thereto.

Another embodiment of the present invention, is to provide a vector comprising the gene expression cassette expressing α-1,2-fucosyl transferase.

Other embodiment of the present invention, is to provide a recombinant *Bacillus* sp. microorganism producing α-1,2-fucosyl transferase, comprising the gene expression cassette.

Other embodiment of the present invention, is to provide a composition for producing 2'-fucosyllactose, comprising at least one selected from the group consisting of α-1,2-fucosyl transferase obtained using the recombinant *Bacillus* sp. microorganism, a microbial cell of the microorganism, a culture of the microorganism, a lysate of the microorganism, and an extract of the lysate or culture.

Other embodiment of the present invention, is to provide a method for producing of 2'-fucosyllactose, comprising a step of culturing a recombinant *Bacillus* sp. microorganism comprising the gene expression cassette.

### [TECHNICAL SOLUTION]

The present invention relates to a gene expression cassette expressing α-1,2-fucosyl transferase in a *Bacillus* sp. microorganism, comprising a nucleic acid sequence encoding α-1,2-fucosyl transferase, and a regulatory sequence operably linked thereto, a recombinant *Bacillus* sp. microorganism comprising the gene expression cassette and a method for producing 2'-fucosyllactose among human milk oligosaccharides using the same, and relates to a method for preparing a recombinant *Bacillus* sp. microorganism by introducing various gene mutations, and optimizing the output of 2'-fucosyllactose of the microorganism. Specifically, the present invention relates to producing 2'-fucosyllactose by selection of enzymes involved in synthesis of 2'-fucosyllactose, ManB, ManC, WcaG, and Gmd, blocking a degradation pathway in order that a strain can use lactose which is a substrate well and overexpression of a membrane transport protein (lacY), selection of a novel constitutive expression promoter and introduction into a *Bacillus* sp. microorganism.

In the present description, "expression cassette" comprises a gene involved in expression of a nucleic acid sequence to be expressed, and the gene expression cassette expressing α-1,2-fucosyl transferase comprises a gene involved in production of α-1,2-fucosyl transferase, and specifically, it comprises a regulatory sequence functionally linked to a coding nucleic acid sequence of α-1,2-fucosyl transferase. Therefore, unlike an expression unit, the expression cassette comprises not only a nucleic acid sequence which regulates transcription and translation, but also a nucleic acid sequence necessary to be expressed as a protein as the result of transcription and translation.

In the present invention, "regulatory sequence" means a nucleic acid which comprises a promoter and has activity of expressing the nucleic acid or the gene, that is, regulating transcription and translation, after being functionally linked to a nucleic acid sequence or gene to be expressed.

In the present description, "promoter", "nucleic acid molecule having promoter activity" or "promoter sequence" means a nucleic acid which is functionally linked to a target nucleic acid sequence to be transcribed and regulates transcription of the target nucleic acid sequence.

The nucleic acid sequence to be transcribed does not necessarily require direct connection to a promoter in a chemical meaning, and may comprise an additional gene regulatory sequence and/or linker nucleic acid sequence, and the like. Arrangement in which the target nucleic acid sequence to be transcribed is located in the downstream of the promoter sequence (i.e., at the 3' end of the promoter sequence) is preferable. The distance between the promoter sequence and nucleic acid sequence to be transcribed may be for example, less than 200 bases, or less than 100 bases.

In the present description, a sequence of "ribosome binding site (RBS)" (or also referred to as Shine-Dalgarno sequence) means an A/G rich polynucleotide sequence.

In the present invention, "operably linked" means a state in which a nucleic acid expression regulatory sequence and a nucleic acid sequence encoding a target protein or peptide are under functional linkage so as to perform general functions. For example, the promoter and nucleic acid sequence encoding a protein or peptide are operably linked, so it may affect expression of a coding sequence. Operable linkage with an expression vector may be prepared using a gene recombination technology well known in the corresponding technical field, and for site-specific DNA cleavage and linkage, enzymes generally known in the corresponding technical field and the like may be used.

All the nucleic acid molecules according to the present invention are preferably nonnaturally occurring, and are nucleic acid molecules in an isolated nucleic acid molecule form or synthesized or prepared by a recombination method. "Isolated" nucleic acid molecule may be removed from other nucleic acid molecules present in natural sources of the nucleic acid, and in addition, when it is prepared by a recombination technology, essentially, other cellular material or culture medium may not be present, or when it is chemically synthesized, a chemical precursor or other chemical substance may not be present.

Hereinafter, the present invention will be described in more detail.

One embodiment of the present invention relates to a gene expression cassette expressing α-1,2-fucosyl transferase in a *Bacillus* sp. microorganism, comprising a nucleic acid sequence encoding α-1,2-fucosyl transferase, and a regulatory sequence operably linked thereto.

The regulatory sequence operably linked to the nucleic acid sequence encoding α-1,2-fucosyl transferase according to one embodiment of the present invention, may be operably linked to the upstream of the nucleic acid sequence encoding α-1,2-fucosyl transferase, comprise a transcription promoter, and additionally comprise a ribosome binding site (RBS).

The regulatory sequence operably linked to the nucleic acid sequence encoding α-1,2-fucosyl transferase according to one embodiment of the present invention may comprise at least one constitutive expression promoter selected from the group consisting of p3610, p12455, p24930, pTu (elongation factor Tu promoter on *Bacillus megaterium* 14581 genome), and pPDC (pyruvate decarboxylase promoter), and the promoter is illustratively described in Table 1 below. The promoter may comprise at least one nucleic acid sequence selected from the group consisting of SEQ ID NOs: 11 to 15. As one embodiment, the promoter may comprise p12455 and/or pTu promoter. As one embodiment, the promoter may comprise a nucleic acid molecule comprising the nucleic acid sequence of SEQ ID NO: 12 and/or SEQ ID NO: 14.

**[Table 1]**

| Name | Sequence (5' to 3' direction) | SEQ ID NO: |
|---|---|---|
| p3610 | CAAATCTTCCTTTCATCTGCTCATATATGATGCTAATTTACGGATAC AACATAAAAAGGCCCATCCGCTCCTAGCATAAGCTAGGGGCGAAT GGACCGCGTTGCCACCCTAATTAGTACACATAATTGTGAACTATCT TTATTCATGTAACGTTGAGGTCAACGTTCAAGCTTTGCACTTGAAA GCTCCAAAGTCCATTCTGCAGGTTTCAATGTTTGTTCTCACCAGCCA CAAACTCTCTGAAAATGAAAATTCTGCATACTACTCTTTTTCACAG | 11 |
| | CTTTAATTATATAGTTATTCGTTATTATAACGCGAAAAAGCAGGAA TTTAAACATCTTATCACTAAAAAGTTTTATAAGACAATTTACTGGG ATTTTTCAGCGGCAAAAAAAGACAAGAACCTTTTAATTAAAGCATT TCTTGATGAAAATTTGGTGGAGGATTTGGTAAAAATCTTTTATTTA ATATTTCTAATGGCTTTGTAATACTATCGTAATACCTATACTACAAA AAAGATTTTAGAAGTCTGACAAGTAGGCACAATCCTTATAAGATAA GGTTTTTTCCCCCATGACAAAAAAATACCATTAAATAATAGTATTG CAATAAATTTTTAAAATATTTATAATAGGCGTAACAAACATATTTT TTACAAAGGTATTACAATTTCTTAATTTTTCAGAAAACACAAAATA GACAATGTTTTGTTTCAATTAGCGAATTTTGTAATGAATGAGTCTTC TACATATTAAGCGCATACATTGGATTTACAGCATGTAAACAATTGA TGACAGTTTCATAGAGTAGAAGAATAATTTTGCAGTGAGGTCTTTA ATGCTCTACTACATGAAAAAATAACTGATTATTTTGTATTGAAAGA GGATTAAGAAATTAGTGCATATCAAAAAAATATGGTTGCCACACA AACAAAATTTAATAGGGGGTAACACGCA | |
| p12455 | AGAAGTTCTCCTTTCCAAATAGGGCTTATAAAATAATTAGCACTTG AGTTGATAGAGTGCTAATTACTCTTCTTATTTAACATGTATTGGGGT ATTTGTCAATATATTAGGAGATTCACCAAAGAGATTAAAAATTAGA CTGAAAAAAGCAAACTTTTAAAAAAAGGTTGAACGTTCTGAAAGA GTTTGCTAGAATATACTTAATTTCAAAGACTATATGTGACTGGCGA AACGCGGATAACCGTGAGGAAGCATATAGTGACGTAGCCGTTCGC CTGGGCAGAGGTAAGGGATTAATACATATCTCTTACCTCTTTCTGTT TAGGGGGAGAGGATATAGAAGTTATCAGACATGTAGGCTGAAAAA CCAGACTTTTAAAAAAATTAAAAATGAAGTTCCTTTATCCAAAGGT AAAAACATGTAAAACTACGATGATAATCAGAGGAGAGTACAAA | 12 |
| p24930 | AAATCTCCACTCCTTTACAGAAAAAATTCCCCATACCTTTCTGTGTA AAACGTGAAAAACAAAATTGAGCCTCTGCTCAACTAGACATTAAA TGATATACGAAGCGCCTCTTTTGAATATATATCTTGGAAGAACATT CCTTCACCCCACGTAAAGAAAATGCTTACATTTCCATTTGTTTCAAT AACACACAAAATCAAAACATCTGCATGTGTCATTGCTTTGTTACAG CCCTATAGGTAAAACATACAATTGTATGTAAAAAGATGCGGCCTCA AAATATATTATAAACAATTATCTGACTTTTGTGAATCAAAAGAGAG AAGAAATTTTAAAAAAATTATTATTATACAAAAATTTCTACGAAAA AAGTTCGGTTAATTTCATAAATAAATAAG | 13 |
| pTu | aatgatgaaggttgaagttgttatccctgacgaatacttaggagatatcatgggtgacattacgtctcgtcgtggac gtgtagaaggtatggaagcacgcggtaacgctcaagttgtacgtgcattcgttccactttctgaaatgtttggttat gcaactgcattacgttcaaacactcaaggtcgcggaacatattctatgcacttcgatcactacgaagaagttccta aatcaatttcagaagaaattattaaaaaaaataaaggtgaataattgattttcactctttattgaagtataactacttat gtacgctgtgaaagtggagttcattccctttcacggcctcataaaacataaactacatgaattttaaggaggattttc ga | 14 |
| pPDC | aggagttaattcatgaaagtggcgatatcactattgtagcaggagctgattatgagttaaaagggaatcaaacatc ttatgtaactgctaaacttccctattccaagcagctgtattccaaaacagtagacttgattcaaatgtcgcgcaatcc aaactctaacaacattcacggtgaattcattggtctttggaaagtttccgcaaaaggcactgaggttgtaaaaaaa gctttggaagaactggcgcaaagaaaagattttgctcagctcacgttttctgatttctttaattatgttaatctaattca cccggttgctgttaaatatacaatggggtcatggatagataccgaaacgtatacaaagattcaaaaagatggtgg gaaaa | 15 |

The constitutive expression promoter may improve the 2'-fucosyllactose productivity by regulating transcription of a nucleic acid sequence encoding α-1,2-fucosyl transferase, which is located in downstream thereof. Specifically, the promoter may increase the 2'-fucosyllactose productivity by 1.05 times or more, 1.1 times or more, 1.2 times or more, 1.3 times or more, 1.4 times or more, 1.45 times or more, 1.5 times or more, 1.55 times or more, or 1.6 times or more, compared to a xylose inducible promoter (pxy1 promoter). The 2'-fucosyllactose productivity may be measured under conditions of a temperature of 30°C and pH 7.0.

The constitutive expression promoter may improve the 2'-fucosyllactose productivity. Specifically, the regulatory sequence may increase the 2'-fucosyllactose productivity by 1.05 times or more, 1.1 times or more, 1.2 times or more, 1.3 times or more, 1.4 times or more, 1.5 times or more, 1.6 times or more, 1.7 times or more, 1.8 times or more, 1.9 times or more, 2 times or more, 2.4 times or more, 3 times or more, 3.4 times or more, or 3.9 times or more, compared to a xylose inducible promoter (pxyl promoter). The 2'-fucosyllactose productivity may be measured under conditions of a temperature of 18°C and pH 7.0.

The constitutive expression promoter may improve the 2'-fucosyllactose productivity at a low temperature. The low temperature may be a temperature of for example, 10 to less than 30°C, 10 to 29°C, 10 to 28°C, 10 to 27°C, 10 to 26°C, 10 to 25°C, 10 to 24°C, 10 to 23°C, 10 to 22°C, 10 to 21°C, 10 to 20°C, 15 to less than 30°C, 15 to 29°C, 15 to 28°C, 15 to 27°C, 15 to 26°C, 15 to 25°C, 15 to 24°C, 15 to 23°C, 15 to 22°C, 15 to 21°C, or 15 to 20°C, for example, 18°C. Specifically, the regulatory sequence may increase the 2'-fucosyllactose productivity at a temperature of 18°C by over 1 time, 1.1 times or more, 1.2 times or more, 1.3 times or more, 1.4 times or more, 1.5 times or more, 1.6 times or more, 1.7 times or more, 1.75 times or more, 1.8 times or more, or 1.85 times or more, compared to the 2'-fucosyllactose productivity at a temperature of 30°C. One specific embodiment of the constitutive expression promoter improving the 2'-fucosyllactose productivity at a low temperature may be p12455, and as one embodiment, it may have the nucleic acid sequence of SEQ ID NO: 12.

The regulatory sequence operably linked to the nucleic acid sequence encoding α-1,2-fucosyl transferase according to one embodiment of the present invention may further comprise a ribosome binding site (RBS), and specifically, it may further comprise an RBS sequence comprising the nucleic acid sequence of SEQ ID NO: 21.

The gene expression cassette according to the present invention expresses α-1,2-fucosyl transferase in a *Bacillus* sp. microorganism, and the nucleic acid sequence encoding α-1,2-fucosyl transferase is not particularly limited, and for example, it may be derived from *Akkermansia muciniphila* or *Bacillus megaterium.* For example, the α-1,2-fucosyl transferase may have at least one amino acid sequence selected from the group consisting of SEQ ID NOs: 1 to 5.

Specifically, the α-1,2-fucosyl transferase according to one embodiment of the present invention is illustratively described in Table 2 below. Accordingly, the nucleic acid sequence encoding α-1,2-fucosyl transferase may comprise a nucleic acid molecule comprising a nucleic acid sequence encoding at least one amino acid sequence selected from the group consisting of SEQ ID NOs: 1 to 5.

**[Table 2]**

| Name | Sequence | SEQ ID NO: |
|---|---|---|
| Am2FT_2 | MNMERKTGLMNKKYVSPCFLPGMRLGNIMFTLAAACAHARTVGV ECRVPWAYNDASLMLRSRLGGWVLPSTPCGTNEPPSWQEPSFAYC PVPSRIRTGGLRGYFQSARYFEGQEAFIRALFAPLTAEKEPGAVGIHI RLGDYRRLRDKHRILDPGFLRRAAGHLSSGKNRLVLFSDEPDEAAE MLARVPAFGRFALEIDRGAPCESLRRMTAMEELVMSCSSFSWWGA WLGNTRKVIVPRDWFVGGVEDYRDIYLPHWVTL | 1 |
| Bm2FT | MKIVQISSGLGNQLFQYALYKRLSMNNNDVFLDVETSYQLNKNQH NGYEIERIFSIQPSHATKGMIDELADVDNRLINRLRRKLFGPKNSMY TETKEFSYDCEVFTKDGIYIKGYWQNYNYFKEIEDDLKNELVFKKA LDLKNSHLINQMNKEISVSIHVRRGDYYLNKEYENKFGNIADLDYY LKAINFIKKEVDDPKFYVFSDDIKWAKENLNLTDNVTYVEHNKGS DSYKDMRLMTCCKHNIIANSTFSWWGAFLNENKNKIVIAPGKWIN VEGVGGINLFPEGWIVY | 2 |
| Bm2FT_2 | MGCIKRLFLYEYGGRCFLVIVKIKGGFGNQLFTYASAYAISQELQQ NLIMDKVIYDLDYFRKFELPSLSLKYDQMLISKFVPNTKVKTLIYKV LRRLKLKGFTEVHEKKEFSFDENIYNLSGDIYLDGYWQNYRYFHK YYKDLSEMFVPRETPRKEVTDYITSLRGVNSVAMHVRRGDYKTFN GGKCLSLDYYIKAMEYFNSNDVQFYVFTDDIDFCEKNLPNSENINY VSRSEKLTDIEEFFIMKECKNFIIANSSFSWWAAYLSEQKADSLIVAP VVDMWKRDFYPDEWVALNTHLE | 3 |
| Bm2FT_3 | MIIVQIKGGLGNQLFSYASAYGIARENETELIIDRYIYDTSYSLRKYM LDFFPEIDEALLLKYIPKKNKISQIMYKLIRKSKLKYKYKAQLFLEEE EFKFTRISTQNENLYLNGYWQSYVVYFDKYRNDIIKKFTPLVSFNQD GNQLLNEIKSYNSVAIHVRRGDYINFKGGKCLDSSYYIKAMKHLYN LKGKNLFFYIFTDDVEYCKRIFKNVANVKFIGEEAKLSDFEEFTLMT HCKNLIIGNSSFSWWAAYLASCKDKAVIAPVVDMWTEDFYLPEWI KIKADLQ | 4 |
| Bs2FT | MKIVHISSGLGNQMFQYALYKKLSLIQDNVFLDTITSYQLYPNQHN GYELEKVFTIKPRHASKELTYNLSDLDNSVTSRIRRKLIGSKKSMYI EHKEFEYDPNLFYQENIYIKGYWQNYDYFKDIENELKNDFTFQRAL DEKNNNLAIKINNENSISIHVRRGDYYLNRKNQEKFGDIANLEYYS KAISYIKERIDNPKFYIFSDNVEWVKQNLNSLEEAVYIDYNVGNDSY KDMQLMSLCKHNIIANSSFSWWGAFLNKNMEKIVIAPGKWINMKG VKKVNLFPKDWIIY | 5 |

For example, the nucleic acid sequence encoding α-1,2-fucosyl transferase may comprise at least one nucleic acid sequence selected from the group consisting of SEQ ID NOs: 6 to 10. Specifically, the nucleic acid sequence encoding α-1,2-fucosyl transferase according to one embodiment of the present invention is illustratively described in Table 3 below.

**[Table 3]**

| Name | Sequence | SEQ ID NO: |
|---|---|---|
| Am2FT_2 nucleic acid sequence | ATGAACATGGAACGGAAAACCGGCCTTATGAATAAAAAATATG TTTCCCCGTGTTTCCTGCCCGGCATGCGGCTGGGCAACATCATGT TCACGCTGGCGGCCGCCTGTGCCCACGCCCGTACCGTAGGGGTG GAGTGCCGGGTGCCCTGGGCTTACAATGACGCTTCCCTGATGCT | 6 |
| | GCGCTCCCGGCTCGGCGGCTGGGTGCTCCCTTCTACGCCGTGCG GCACCAATGAACCTCCATCCTGGCAGGAACCTTCCTTTGCCTATT GTCCGGTTCCCTCCCGCATCCGGACGGGCGGGCTTCGCGGCTAC TTCCAGAGCGCACGGTATTTTGAAGGGCAGGAAGCGTTCATCCG GGCGCTTTTCGCGCCTCTCACCGCGGAGAAGGAGCCCGGCGCCG TTGGCATTCACATCCGGCTTGGGGACTACAGGCGCCTCCGTGAC AAGCACCGCATTCTGGACCCTGGTTTCCTGCGGAGGGCCGCAGG GCATTTATCATCCGGGAAGAACCGTCTGGTGCTGTTCAGCGATG AGCCGGACGAGGCGGCGGAGATGCTTGCGCGCGTGCCGGCATT CGGACGCTTTGCGCTGGAAATCGACCGCGGCGCTCCGTGCGAAT CCCTCCGCCGCATGACGGCGATGGAGGAACTCGTCATGTCGTGT TCTTCCTTTTCCTGGTGGGGCGCGTGGCTGGGAAATACCCGGAA GGTCATCGTTCCGCGCGACTGGTTTGTCGGCGGTGTGGAAGACT ACCGGGATATTTACCTGCCCCATTGGGTAACACTGTAA | |
| Bm2FT nucleic acid sequence | ATGAAAATTGTTCAGATTTCTTCTGGTCTGGGTAACCAGCTGTTC CAGTACGCCCTGTACAAACGTCTGTCTATGAACAACAACGATGT GTTCCTGGATGTGGAGACGTCTTATCAACTGAACAAAAATCAGC ACAATGGTTACGAAATTGAACGTATTTTTTCCATCCAGCCATCCC ATGCTACCAAGGGTATGATCGATGAACTGGCCGATGTAGACAAC CGTCTGATCAACCGCTTACGTCGTAAACTGTTTGGTCCGAAAAA CTCTATGTATACCGAAACCAAAGAGTTCAGCTACGATTGTGAAG TTTTCACCAAAGATGGTATCTATATCAAGGGCTACTGGCAGAAC TATAACTATTTTAAAGAGATTGAAGACGATCTGAAAAATGAACT GGTCTTTAAAAAAGCTCTGGATCTGAAAAATTCGCACCTGATCA ATCAGATGAATAAAGAAATTAGCGTAAGCATCCACGTACGCCGC GGCGATTATTACCTGAACAAGGAGTACGAAAACAAATTTGGCA ACATTGCGGATCTTGATTATTATCTGAAAGCGATTAACTTCATCA AAAAAGAAGTAGACGATCCCAAATTTTACGTTTTCTCCGACGAT ATTAAATGGGCTAAAGAAAATCTGAACCTGACGGATAACGTAA CTTATGTTGAACACAACAAAGGGAGCGACTCTTACAAAGACATG CGTCTGATGACCTGCTGTAAACATAACATCATCGCCAACTCTAC ATTCTCTTGGTGGGGCGCGTTTCTTAACGAGAACAAGAACAAAA TCGTAATCGCGCCGGGCAAATGGATCAACGTTGAAGGCGTCGGT GGCATCAACCTGTTCCCTGAAGGTTGGATCGTTTACTGA | 7 |
| Bm2FT_2 nucleic acid sequence | ATG GGT TGC ATA AAA AGA CTA TTC TTA TAT GAA TAC GGA GGA CGA TGT TTT TTG GTA ATT GTT AAA ATA AAA GGT GGA TTT GGT AAC CAA TTG TTT ACA TAC GCT AGT GCT TAT GCA ATA TCT CAA GAA CTG CAA CAA AAC TTA ATT ATG GAT AAG GTA ATA TAT GAT CTA GAT TAC TTT AGA AAA TTT GAA TTA CCT TCA TTA TCG TTG AAG TAT GAT CAA ATG TTA ATT AGT AAG TTT GTA CCT AAT ACT AAG GTT AAA ACT TTA ATT TAT AAA GTT TTA AGG AGG TTA AAA TTA AAA GGA TTT ACA GAG GTA CAT GAA AAA AAG GAA TTT TCT TTT GAT GAA AAT ATT TAC AAT TTG TCA GGT GAT ATT TAT TTA GAT GGT TAT TGG CAG AAT TAT AGA TAT TTC CAT AAA TAT TAT AAA GAT CTG TCG GAA ATG TTT GTA CCA AGA GAA ACT CCT AGA AAA GAA GTA ACT GAT TAT ATC ACG TCT TTA CGG GGA GTT AAT TCT GTT GCT ATG CAT GTT AGG AGA GGA GAT TAT AAA ACT TTT | 8 |
| | AAC GGA GGA AAA TGT TTG TCT TTA GAT TAT TAT ATT AAG GCT ATG GAA TAT TTC AAT TCA AAT GAT GTA CAA TTT TAT GTA TTT ACA GAT GAT ATT GAT TTT TGT GAG AAA AAC CTC CCG AAT AGT GAA AAT ATT AAT TAT GTG TCA AGA AGC GAG AAA CTT ACA GAT ATT GAA GAA TTC TTT ATT ATG AAA GAA TGT AAA AAT TTT ATA ATT GCT AAT AGT AGT TTT TCG TGG TGG GCT GCT TAT TTA AGT GAA CAG AAA GCA GAT AGT TTA ATT GTA GCC CCT GTT GTT GAC ATG TGG AAA AGA GAT TTT TAT CCA GAT GAG TGG GTG GCA TTG AAT ACA CAT TTA GAA TAG | |
| Bm2FT_3 nucleic acid sequence | ATG ATA ATT GTT CAA ATC AAG GGC GGA TTA GGT AAT CAA CTT TTT TCG TAT GCT TCT GCT TAT GGA ATT GCA AGA GAG AAT GAA ACA GAA TTA ATT ATT GAT CGA TAT ATA TAT GAT ACA AGC TAC TCT TTA AGA AAA TAT ATG TTG GAT TTT TTC CCT GAG ATC GAT GAA GCA CTA CTT CTA AAG TAT ATA CCT AAA AAA AAT AAA ATT TCA CAA ATT ATG TAT AAA TTG ATT AGA AAA TCT AAG TTG AAA TAT AAG TAC AAA GCT CAA TTA TTT TTA GAA GAA GAG GAA TTT AAG TTT ACT AGA ATT AGT ACC CAG AAT GAA AAT TTG TAT TTA AAT GGT TAT TGG CAA AGT TAT GTG TAC TTT GAT AAA TAT CGT AAT GAC ATT ATT AAA AAA TTC ACC CCT TTA GTA AGC TTT AAT CAA GAT GGA AAT CAA CTT CTT AAC GAA ATT AAA AGT TAT AAT TCA GTG GCT ATC CAT GTC AGG AGA GGG GAT TAT ATT AAT TTT AAA GGT GGA AAG TGT TTA GAT AGT TCT TAT TAT ATA AAG GCA ATG AAG CAT TTA TAC AAT TTA AAA GGA AAA AAT TTG TTT TTT TAT ATT TTT ACA GAT GAT GTG GAA TAT TGT AAA AGA ATT TTT AAA AAC GTA GCT AAT GTT AAA TTT ATA GGT GAG GAA GCA AAA TTG AGT GAT TTT GAA GAG TTT ACT TTG ATG ACT CAT TGT AAA AAC CTC ATA ATA GGT AAC AGC TCA TTT TCT TGG TGG GCA GCT TAC TTA GCC TCT TGT AAA GAT AAA GCT GTA ATA GCT CCA GTG GTA GAT ATG TGG ACT GAA GAT TTC TAT CTA CCT GAA TGG ATA AAA ATT AAA GCG GAT TTA CAA TAG | 9 |
| Bs2FT nucleic acid sequence | ATG AAA ATA GTA CAC ATA AGT AGC GGT TTA GGA AAT CAA ATG TTT CAA TAT GCT CTA TAT AAA AAA CTG TCC TTA ATT CAA GAT AAT GTT TTT TTG GAT ACA ATT ACC AGT TAT CAG TTG TAT CCT AAT CAA CAT AAT GGA TAT GAG TTA GAG AAG GTA TTT ACA ATT AAA CCA AGG CAT GCA AGT AAA GAA TTA ACA TAC AAT TTA TCA GAC TTA GAT AAT TCA GTA ACA TCT CGC ATT CGT AGG AAA TTA ATT GGA AGT AAA AAG AGC ATG TAT ATA GAA CAT AAA GAA TTT GAA TAC GAT CCT AAT TTA TTT TAT CAA GAA AAT ATA TAT ATA AAA GGT TAC TGG CAG AAT TAT GAT TAT TTT AAA GAT ATA GAA AAT GAA TTA AAG AAT GAT TTT ACT TTT CAA AGA GCT TTA GAT GAG AAA AAT AAT AAT TTA GCT ATT AAA ATT AAC AAT GAG AAT TCA ATA TCA ATT CAT GTA AGA CGT GGT GAT TAT TAC TTA AAC AGA AAG AAT CAA GAG AAG TTT GGA GAT ATC GCT AAT TTG GAA TAC TAT TCA AAG GCA ATT AGC TAT ATT AAA GAA AGA ATA | 10 |
| | GAC AAT CCA AAG TTT TAT ATC TTT TCA GAT AAT GTT GAG TGG GTA AAG CAA AAT CTT AAT TCG TTA GAG GAA GCT GTA TAT ATA GAT TAT AAC GTG GGA AAT GAT AGT TAC AAG GAT ATG CAA CTA ATG AGT CTT TGT AAA CAT AAT ATT ATT GCT AAT AGT TCT TTT AGT TGG TGG GGA GCA TTT TTG AAT AAG AAT ATG GAG AAA ATA GTT ATT GCA CCT GGG AAG TGG ATT AAT ATG AAA GGT GTA AAA AAA GTC AAT CTT TTC CCA AAG GAT TGG ATA ATT TAT TAA | |

The gene expression cassette expressing α-1,2-fucosyl transferase according to one embodiment of the present invention may further comprise a nucleic acid sequence encoding a lactose membrane transport protein (lactose permease). The lactose membrane transport protein may be derived from *Kluyveromyces lactis* or *Bacillus megaterium.*

For example, the lactose membrane transport protein may be Lac12 and/or LacY. Specifically, the lactose membrane transport protein may comprise an amino acid sequence of SEQ ID NO: 17 and/or SEQ ID NO: 18. More specifically, the Lac12 may have SEQ ID NO: 17, and the LacY may have SEQ ID NO: 18.

For example, the nucleic acid sequence encoding the lactose membrane transport protein may comprise a nucleic acid sequence of SEQ ID NO: 19 and/or SEQ ID NO: 20. More specifically, the nucleic acid sequence encoding Lac12 may comprise the nucleic acid sequence of SEQ ID NO: 19, and the nucleic acid sequence encoding LacY may comprise the nucleic acid sequence of SEQ ID NO: 20.

The lactose membrane transport protein may improve the 2'-fucosyllactose productivity. Specifically, the gene expression cassette further comprising a nucleic acid sequence encoding the lactose membrane transport protein, may increase the 2'-fucosyllactose productivity by 1.1 times or more, 1.2 times or more, 1.25 times or more, or 1.3 times or more compared to a gene expression cassette not comprising nucleic acid sequence encoding the lactose membrane transport protein. The 2'-fucosyllactose productivity may be measured under conditions of a temperature of 30°C and pH 7.0.

As one specific embodiment, the lactose membrane transport protein is LacY, and may improve the 2'-fucosyllactose productivity. Specifically, the gene expression cassette comprising a nucleic acid sequence encoding LacY, may increase the 2'-fucosyllactose productivity by 1.05 times or more, 1.1 times or more, 1.14 times or more, or 1.15 times or more, compared to a gene expression cassette comprising a nucleic acid sequence encoding Lac12. The 2'-fucosyllactose productivity may be measured under conditions of a temperature of 30°C and pH 7.0.

The gene expression cassette expressing α-1,2-fucosyl transferase according to one embodiment of the present invention may further comprise a regulatory sequence of a nucleic acid sequence encoding the lactose membrane transport protein, and the regulatory sequence of the nucleic acid sequence encoding the lactose membrane transport protein may be a promoter and/or an RBS. For example, the regulatory sequence of the nucleic acid sequence encoding the lactose membrane transport protein may comprise a ribosome binding site (RBS) comprising the nucleic acid sequence of SEQ ID NO: 22.

According to one specific embodiment of the present invention, the *Bacillus* sp. microorganism may be at least one selected from the group consisting of *Bacillus megaterium, Bacillus subtilis, Bacillus cereus, Bacillus licheniformis, Bacillus amyloliquefaciens,* and *Bacillus thuringiensis,* and as one embodiment, it may be *Bacillus megaterium,* but not limited thereto.

The gene expression cassette expressing α-1,2-fucosyl transferase according to one embodiment of the present invention may comprise a transcription promoter, an RBS, and a nucleic acid molecule encoding α-1,2-fucosyl transferase, located sequentially in the direction from 5' end to 3' end. Preferably, the gene expression cassette may comprise a transcription promoter, an RBS, a nucleic acid molecule encoding α-1,2-fucosyl transferase, and a nucleic acid sequence encoding a lactose membrane transport protein, located sequentially in the direction from 5' end to 3' end. More preferably, the gene expression cassette may comprise a promoter, an RBS, a nucleic acid molecule encoding α-1,2-fucosyl transferase, a regulatory sequence of a nucleic acid sequence encoding a lactose membrane transport protein, and a nucleic acid sequence encoding a lactose membrane transport protein, located sequentially in the direction from 5' end to 3' end. The promoter, the RBS, the lactose membrane transport protein and the like are as described above.

In one embodiment of the present invention, the expression cassette expressing α-1,2-fucosyl transferase may further comprise at least one sequence selected from the group consisting of a replication starting point, a leader, a selectable marker, a cloning stie, and a restriction enzyme recognition site, as a polynucleotide sequence for expression.

Other one embodiment of the present invention relates to a vector, particularly, a shuttle vector or plasmid vector, comprising the gene expression cassette expressing α-1,2-fucosyl transferase according to the present invention. The gene expression cassette expressing α-1,2-fucosyl transferase is as described above.

The recombinant vector according to one embodiment of the present invention may be constructed as a vector for cloning or a vector for expression by a method widely known in the corresponding technical field. For the recombinant vector, any vector used for gene recombination may be used, and for example, it may be selected from the group consisting of plasmid expression vectors, virus expression vectors (e.g., replication defective retrovirus, adenovirus, and adenoassociated virus) and virus vectors capable of performing the equivalent function thereto, but not limited thereto.

For example, the recombinant vector may be constructed from those selected from the group consisting of pMM1525, pHIS1525, pSTOP1622, and pMGBm19 vectors, and the like, and for example, it may be P3stop1623 2RBS vector.

The polynucleotide of the gene expression cassette may be used as it is, or in a form of a recombinant vector comprising the polynucleotide. The recombinant vector means a recombinant nucleic acid molecule which can deliver an operably linked target polynucleotide, and the target polynucleotide may be operably linked to at least one transcription regulatory element consisting of a promoter and a transcription termination factor, and the like.

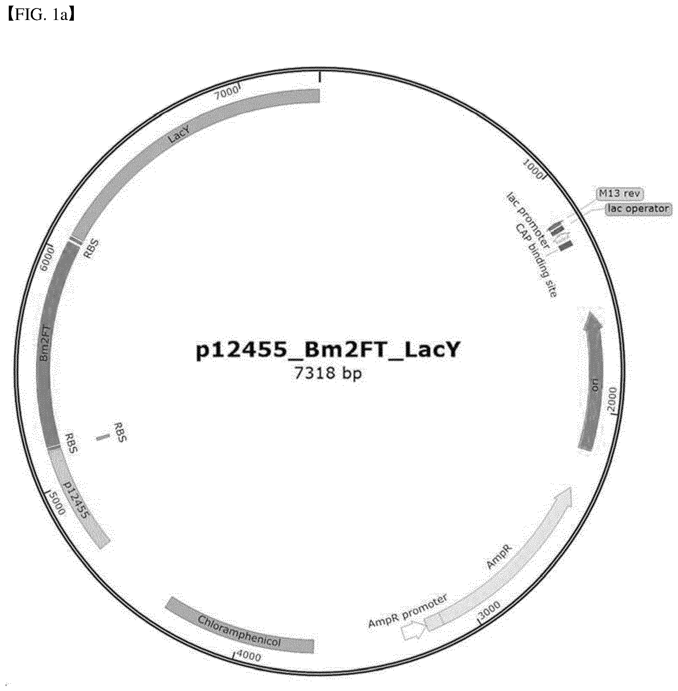

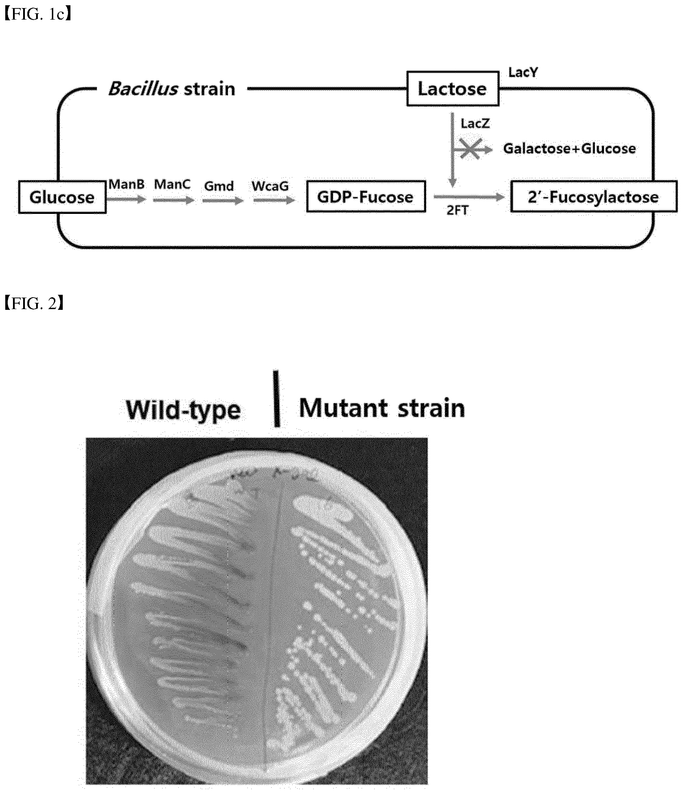

As one embodiment, the vector may be a recombinant vector having the cleavage map of FIG. 1a.

According to one embodiment of the present invention, the vector may further comprise a fucose synthesis gene expression cassette in addition to the expression cassette expressing α-1,2-fucosyl transferase.

The fucose synthesis gene expression cassette may comprise a nucleic acid sequence encoding a fucose synthesis gene, and a regulatory sequence operably linked thereto.

Specifically, the fucose synthesis gene expression cassette may comprise at least one selected from the group consisting of a nucleic acid sequence encoding GTP mannose-1-phosphate guanylyltransferase (ManC), a nucleic acid sequence encoding phosphomannomutase (Hypo or MnB), a nucleic acid sequence encoding GDP-L-fucose synthase (WcaG), and a nucleic acid sequence encoding GDP-D-mannose-4,6-dehydratase (Gmd), and a regulatory sequence operably linked thereto. Preferably, the fucose synthesis gene expression cassette may comprise GTP mannose-1-phosphate guanylyltransferase (ManC).

For example, the fucose synthesis gene expression cassette may comprise a nucleic acid sequence encoding at least one amino acid sequence selected from the group consisting of SEQ ID NOs: 23 to 26.

For example, the fucose synthesis gene expression cassette may comprise at least one nucleic acid sequence selected from the group consisting of SEQ ID NOs: 27 to 30.

The regulatory sequence operably linked to the nucleic acid sequence encoding the fucose synthesis gene, may be operably linked to the upstream of the fucose synthesis gene, and comprise a transcription promoter, and may additionally comprise a ribosome binding site (RBS).

For example, the regulatory sequence operably linked to the nucleic acid sequence encoding the fucose synthesis gene, may comprise at least one constitutive expression promoter selected from the group consisting of p3610, p12455, p24930, p0706, pTu, and pPDC. For example, the regulatory sequence may comprise at least one selected from the group consisting of SEQ ID NOs: 11 to 16. As one embodiment, the regulatory sequence may comprise p12455 and/or p0706 promoters. Specifically, the regulatory sequence may comprise a nucleic acid molecule comprising the nucleic acid sequence of SEQ ID NO: 12 and/or SEQ ID NO: 16.

The regulatory sequence operably linked to the nucleic acid sequence encoding the fucose synthesis gene, may further comprise a ribosome binding site (RBS) comprising the nucleic acid sequence of SEQ ID NO: 31.

Other one embodiment of the present invention relates to a recombinant microorganism, in particular, *Bacillus* sp. microorganism, comprising a vector, in particular, shuttle vector or plasmid vector, which delivers at least one gene expression cassette according to the present invention. In other words, other one embodiment of the present invention relates to a recombinant *Bacillus* sp. microorganism comprising the gene expression cassette according to the present invention, or transformed with a vector comprising the expression cassette.

For the method for transforming a host cell with the recombinant vector, all transformation methods known in the art may be selected and used without limitation, and for example, it may be selected from fusion of bacterial protoplasts, electroporation, projectable bombardment, and infection using a virus vector, and the like.

The recombinant *Bacillus* sp. microorganism according to one embodiment of the present invention can overexpress introduced α-1,2-fucosyl transferase, and therefore, it can provide the high stable α-1,2-fucose converting activity for a long period of time. Therefore, the recombinant *Bacillus* sp. microorganism may be useful in preparation of α-1,2-fucose, and the α-1,2-fucose producing yield may be enhanced.

The recombinant *Bacillus* sp. microorganism according to one embodiment of the present invention may have fucose synthesis activity, or may have an improved activity for fucose synthesis. Specifically, the recombinant microorganism according to one embodiment of the present invention may be characterized by having fucose synthase, for example, at least one selected from the group consisting of GDP-D-mannose-4,6-dehydratase, GDP-L-fucose synthase (WcaG), phosphomannomutase, and GTP-mannose-1-phosphate guanylyltransferase.

As one specific embodiment, the recombinant *Bacillus* sp. microorganism with improved fucose synthesis activity may have been introduced with GDP-D-mannose-4,6-dehydratase, GDP-L-fucose synthase (WcaG), phosphomannomutase, and GTP-mannose-1-phosphate guanylyltransferase.

As one specific embodiment, the recombinant *Bacillus* sp. microorganism with improved fucose synthesis activity may have been introduced with GTP mannose-1-phosphate guanylyltransferase (ManC), and may have not been introduced with phosphomannomutase (Hypo).

As one specific embodiment, the recombinant *Bacillus* sp. microorganism with improved fucose synthesis activity may have been introduced with phosphomannomutase (Hypo), and may have not been introduced with GTP mannose-1-phosphate guanylyltransferase (ManC).

As one specific embodiment, the recombinant *Bacillus* sp. microorganism with improved fucose synthesis activity may have been introduced with GDP-L-fucose synthase (WcaG), and may have not been introduced with GDP-D-mannose-4,6-dehydratase (Gmd).

As one specific embodiment, the recombinant *Bacillus* sp. microorganism with improved fucose synthesis activity may have been introduced with GDP-D-mannose-4,6-dehydratase (Gmd), and may have been further introduced with phosphomannomutase and/or GTP-mannose-1-phosphate guanylyltransferase.

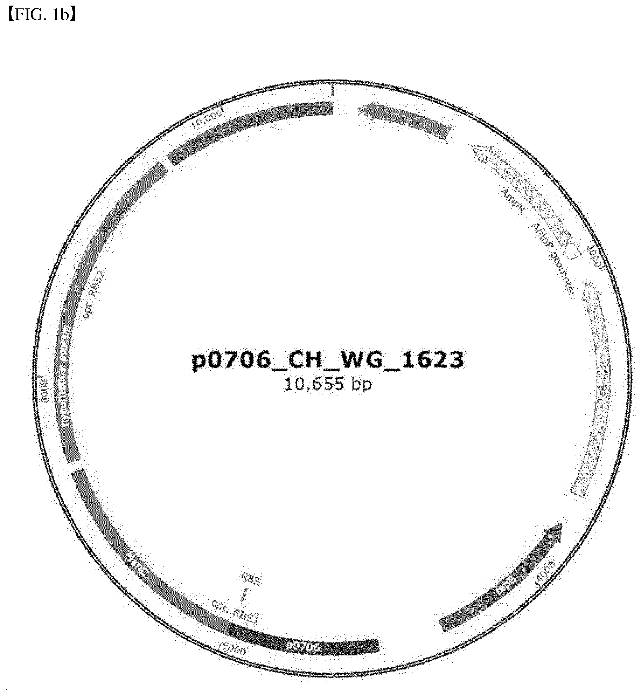

Specifically, the recombinant *Bacillus* sp. microorganism may have an improved activity for fucose synthesis as the vector to be introduced into the recombinant *Bacillus* sp. microorganism comprises a fucose synthesis gene expression cassette, or as the recombinant *Bacillus* sp. microorganism further comprises a vector comprising a fucose synthesis gene expression cassette. In other words, the recombinant *Bacillus* sp. microorganism according to one embodiment of the present invention may further comprise a separate vector comprising a gene expression cassette expressing a fucose synthesis gene, in addition to a vector comprising the gene expression cassette expressing α-1,2-fucosyl transferase according to the present invention. As one embodiment, the vector comprising the fucose synthesis gene expression cassette may be a recombinant vector having the cleavage map of FIG. 1b.

Accordingly, the recombinant *Bacillus* sp. microorganism according to one embodiment of the present invention has improved activity for fucose synthesis in addition to the characteristic of overexpressing α-1,2-fucosyl transferase, and the α-1,2-fucose production yield can be further enhanced.

Specifically, the recombinant *Bacillus* sp. microorganism having the fucose synthesis activity, or with improved fucose synthesis activity, may increase the 2'-fucosyllactose productivity by 1.05 times or more, 1.1 times or more, 1.15 times or more, or 1.18 times or more, compared to a control group having no fucose synthesis activity. The control group having no fucose synthesis activity may be for example, a control group not overexpressing a fucose synthesis gene, or a control group not being introduced a fucose synthesis gene. As one embodiment, the control group may be a ΔLacZ mutant strain which expresses a lactose membrane transport protein, and fucosyl transferase linked to a constitutive expression promoter is introduced into, but a fucose synthesis gene is not introduced into.

The recombinant *Bacillus* sp. microorganism according to one embodiment of the present invention may be one in which the activity of lactase is reduced or removed. Specifically, the microorganism may be one in which LacZ gene is removed.

According to one embodiment of the present invention, the Bacillus sp. microorganism may be at least one selected from the group consisting of *Bacillus megaterium, Bacillus subtilis, Bacillus cereus, Bacillus licheniformis, Bacillus amyloliquefacien,* and *Bacillus thuringiensis,* and as one embodiment, it may be *Bacillus megaterium,* but not limited thereto.

Other one embodiment of the present invention, relates to a composition for producing 2'-fucosyllactose, comprising at least one selected from the group consisting of α-1,2-fucosyl transferase obtained by using the recombinant *Bacillus* sp. microorganism, a microbial cell of the microorganism, a culture of the microorganism, a lysate of the microorganism, and an extract of the lysate or culture. The recombinant *Bacillus* sp. microorganism, the α-1,2-fucosyl transferase, the 2'-fucosyllactose, and the like are as described above.

The culture comprises an enzyme produced from the *Bacillus* sp. microorganism, and it may comprise the microorganism, or may be in a cell-free form not comprising the microorganism. The lysate means a lysate obtained by lysing the *Bacillus* sp. microorganism or a supernatant obtained by centrifuging the lysate, and comprises an enzyme produced from the *Bacillus* sp. microorganism. In the present description, unless otherwise mentioned, the *Bacillus* sp. microorganism used in preparation of 2'-fucosyllactose is used to mean at least one selected from the group consisting of a microbial cell of the microorganism, a culture of the microorganism and a lysate of the microorganism.

Other one embodiment of the present invention relates to a method for producing 2'-fucosyllactose, comprising a step of culturing a recombinant *Bacillus* sp. host cell, which comprises the gene expression cassette according to the present invention, or is transformed with a vector comprising the expression cassette. The gene expression cassette, the vector comprising the expression cassette, the recombinant *Bacillus* sp. host cell, and the like are as described above.

As one specific embodiment, the gene expression cassette may comprise a constitutive expression promoter of p12455 as a regulatory sequence, and improve the 2'-fucosyllactose productivity in low temperature. As one embodiment, the regulatory sequence may comprise a constitutive expression promoter of p12455, and the step of culturing may be culturing at a temperature of 10 to 25°C.

The method for producing may produce 2'-fucosyllactose by reacting at least one selected from the group consisting of α-1,2-fucosyl transferase obtained from the culture, a microbial cell of the microorganism, a culture of the microorganism, a lysate of the microorganism, and an extract of the lysate or culture, with a lactose-containing raw material.

### [ADVANTAGEOUS EFFECTS]

The present invention can produce 2'-fucosyllactose by using a GRAS (Generally Recognized As Safe) strain with biosafety level 1, and can provide a recombinant *Bacillus* sp. microorganism having excellent productivity of 2'-fucosyllactose without consumer concern about stability, and a method for producing 2'-fucosyllactose using the same.

### [BRIEF DESCRIPTION OF THE DRAWINGS]

FIG. 1a and FIG. 1b are vector cleavage maps according to an embodiment of the present invention.
FIG. 1c is a drawing which shows the 2'-fucosyllactose biosynthesis pathway of the recombinant microorganism according to an embodiment of the present invention.
FIG. 2 is a drawing which shows the result of comparing the phenotypes of the wild type and ΔLacZ mutant strain through X-gal experiment according to an embodiment of the present invention.
FIG. 3 is a drawing which shows the result of comparing the amount of remaining lactose in the ΔLacZ mutant strain and wild type over time according to an embodiment of the present invention.
FIG. 4 is a drawing which shows the 2'-fucosyllactose productivity effect according to introduction of a constitutive expression promoter into the ΔLacZ mutant strain according to an embodiment of the present invention.
FIG. 5 is a drawing which shows the producing effect of 2'-fucosyllactose according to introduction of Lac12 into the ΔLacZ mutant strain according to an embodiment of the present invention.
FIG. 6 is a drawing which shows the producing effect of 2'-fucosyllactose according to introduction of LacY into the ΔLacZ mutant strain according to an embodiment of the present invention.
FIG. 7 is a drawing which shows the result of comparing productivity through combination of various GDP-fuc synthesis enzymes into the ΔLacZ mutant strain according to an embodiment of the present invention.
FIG. 8 is a drawing which shows the result of comparing production of 2'-fucosyllactose for each combination strain of various GDP-fuc enzymes and constitutive expression promoters into the ΔLacZ mutant strain according to an embodiment of the present invention.

### [MODE FOR INVENTION]

Hereinafter, the present invention will be described in more detail by the following Examples. However, these Examples are intended to illustrate the present invention only, but the scope of the present invention is not limited by these Examples.

### Example 1. Preparation of Bacillus sp. microorganism in which lactase is removed

### 1-1: Preparation of ΔLacZ mutant strain

*Bacillus megaterium* 14581 selected for preparing microorganism in the present invention was obtained from Korean Culture Center of Microorganisms (KCCM) as Accession number KCCM 40441.

β-galactosidase (hereinafter, LacZ) gene in the genome of *Bacillus megaterium* 14581 was deleted by a homologous recombination method, and the deletion of the gene was verified by genome sequencing again.

### 1-2: Analysis of LacZ deletion through phenotype

In order to test whether the phenotype of the obtained ΔLacZ *bacillus megaterium* 14581 strain was changed, an x-gal experiment was performed. As shown in in Fig. 2, the wild type had a blue color as it retained the LacZ gene and degraded x-gal, whereas the mutant strain of ΔLacZ *bacillus megaterium* 14581 prepared in the present Example maintained an original white color of the strain as it did not degrade x-gal.

### 1-3: Analysis of LacZ deletion through lactose degrading activity

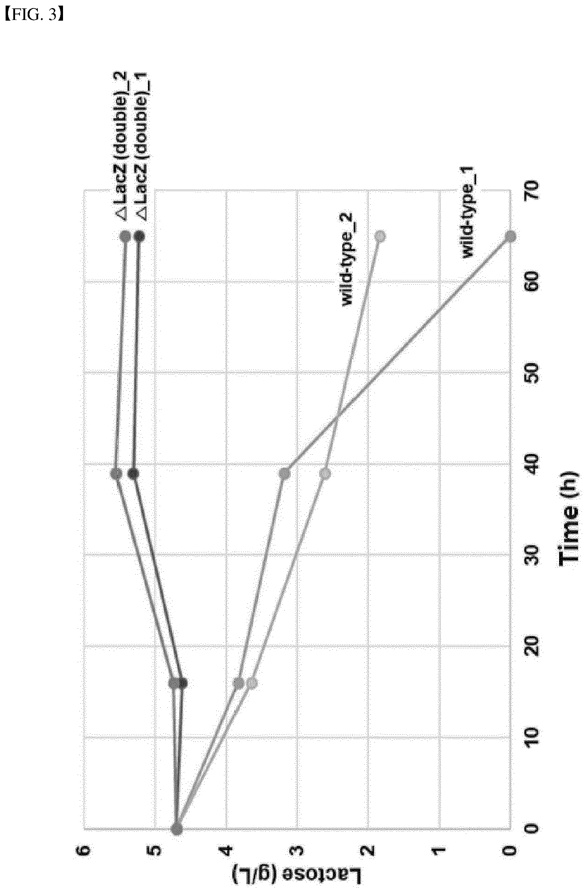

In order to observe the degree of lactose degradation of the mutant strain, the change in the lactose amount over time by adding lactose into the prepared ΔLacZ mutant strain was analyzed through HPLC analysis. As shown in FIG. 3, it was confirmed that the prepared ΔLacZ mutant strain maintained the residual amount of lactose, unlike the wild type.

### Example 2. Search for constitutive expression promoter and analysis of temperature characteristic in ΔLacZ_mutant strain

### 2-1: Screening for fucosyl transferase

*Bacillus megaterium* 14581 to be produced does not have fucosyl transferase and cannot produce 2'fucosyllatose. Therefore, fucosyl transferase was to be artificially introduced, and five kinds of α-1,2-fucosyl transferase derived from the Biosafety level 1 strain were selected as enzymes. The selected fucosyl transferase are shown in Table 2. In Table 2, fucosyl transferase of SEQ ID NO: 1 was derived from *Akkermansia muciniphila,* and named Am2FT_2. The fucosyl transferases of SEQ ID NOs: 2 to 4 were derived from *Bacillus megaterium,* and named Bm2FT, Bm2FT_2, and Bm2FT_3, respectively. The fucosyl transferase of SEQ ID NO: 5 was derived from *Bacillus* sp., and named Bs2FT.

In order to test the 2'-fucosyllactose producing activity of the selected five enzymes, *Bacillus megaterium* was transformed by the plasmids for the five enzymes, respectively, and then grown at 37°C. Two colonies were inoculated to each plate to perform overnight seed culture. Seed 200 uL was input to a 4mL medium added with 22mM and reacted at 30°C. After 20 hours, it was analyzed by HPLC. As a result of testing the 2'-fucosyllactose-producing activity, Am2FT_2 produced about 5 (mg/L), Bm2FT produced about 15 (mg/L), Bm2FT_2 produced about 3 (mg/L), Bm2FT_3 produced about 4 (mg/L), and Bs2FT produced about 1 (mg/L) of 2'-fucosyllactose, and all five enzymes had the 2FL-producing activity. Among them, the Bm2FT enzyme was representatively selected and used for a subsequent experiment.

### 2-2: Search of constitutive expression promoter

A conventional promoter used mainly for overexpressing a recombinant protein in *Bacillus megaterium* is a xylose inducible promoter (hereinafter, pXyl). In order to overexpress a protein without addition of xylose, a constitutive expression promoter was searched on *Bacillus megaterium* 14581 genome. In order to overexpress the Bm2FT gene in a vector form, constitutive expression promoters of p3610, p12455, and p24930 instead of the xylose inducible promoter were finally selected and plasmids were constructed to express Bm2FT, and transformed into the LacZ deleted strain prepared in Example 1. Final production of 2'-fucosyllactose was analyzed through HPLC method.

Specifically, 22mM lactose was added until OD₆₀₀ was about ~0.8, and then, it was analyzed by HPLC after 20 hours. In case of the five test tube reaction, seed 200 uL was input to a 4mL medium added with 22mM and then analyzed by HPLC.

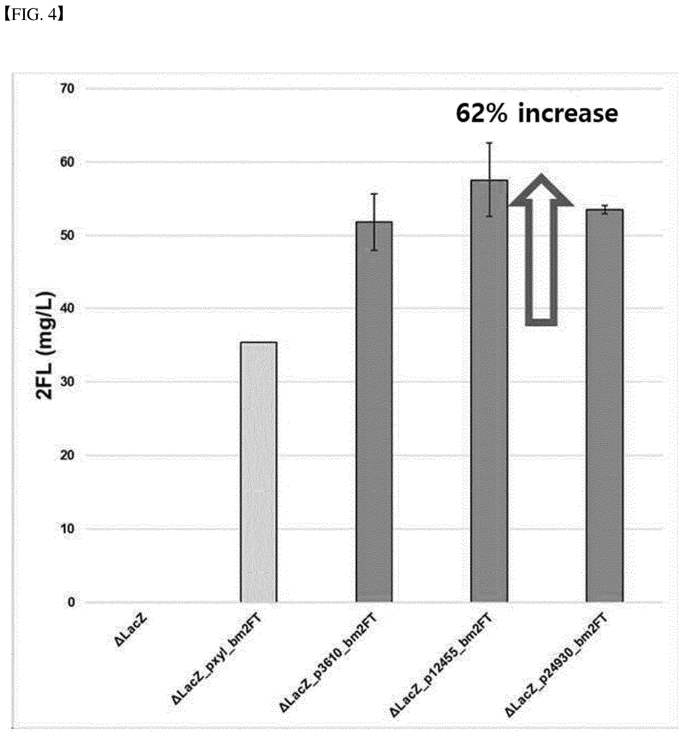

The effect of introduction of the constitutive expression promoter of the present invention on 2'-fucosyllactose productivity are shown in Table 4 and FIG. 4. When three kinds of the constitutive expression promoters were introduced, all of them showed the increased productivity of 2'-fucosyllactose compared to conventional pxyl at pH 7.0 and a temperature of 30°C, and in particular, the productivity of p12455 was increased by 60% or more compared to pXyl.

**[Table 4]**

| Classification | 2FL (mg/L), 30°C |
|---|---|
| ΔLacZ | 0 |
| ΔLacZ_pxy1_bm2FT | 35.4 |
| ΔLacZ_p3610_bm2FT | 51.8 |
| ΔLacZ_p12455_bm2FT | 57.6 |
| ΔLacZ_p24930_bm2FT | 53.5 |

### 2-3: Evaluation of productivity at low temperature

In order to test the change in the productivity of each promoter at a low temperature, five strains in which the constitutive expression promoter was introduced with Bm2FT were cultured at a temperature of 18°C for 65 hours, respectively, and then production of 2'-fucosyllactose over time was analyzed through HPLC method, and the amount (mg/L) of produced 2FL was shown in Table 5. As shown in Table 5, the productivity of p12455 was increased by about 1.8 times or more at a low temperature.

**[Table 5]**

| Classification | 2FL (mg/L), 18°C |
|---|---|
| ΔLacZ | 0 |
| ΔLacZ_pxy1_bm2FT | 30.7 |
| ΔLacZ_p3610_bm2FT | 34.4 |
| ΔLacZ_p12455_bm2FT | 107 |
| ΔLacZ_p24930_bm2FT | 38.3 |

### Example 3. Search of constitutive expression promoter in ΔLacZ mutant strain expressing lactose membrane transport protein

### 3-1: Introduction of Lac12

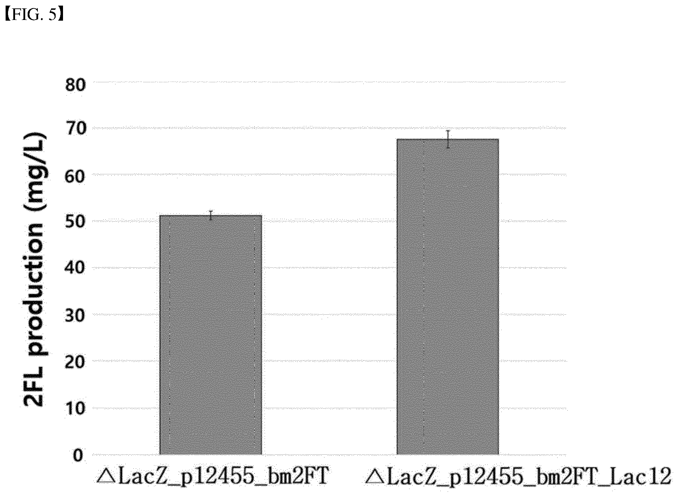

In order to transport more lactose into cells, a lactose membrane transport protein derived from *Kluyveromyces lactis* (hereinafter, Lac12) was introduced into ΔLacZ *Bacillus megaterium* prepared in Example 2 that the constitutive expression promoter p12455 was introduced, and effect thereof was analyzed. The Lac12 and Bm2FT were introduced polycistronically as a plasmid form into the strain and the strain was cultured at a temperature of 30°C and pH 7, and then produced amount thereof after 65 hours was analyzed and compared with the strain introduced with only Bm2FT, to show in Table 6 and FIG. 5. As shown in Table 6 and FIG. 5, it was confirmed that in case of introducing Lac12 together with Bm2FT, the amount of produced 2FL was increased by 30% or more, compared to that of introduction of only Bm2FT.

**[Table 6]**

| Classification | 2FL (mg/L), 30°C, 65h |
|---|---|
| ΔLacZ_p12455 _bm2FT | 51.3 |
| ΔLacZ_p12455_bm2FT_Lac12 | 67.6 |

### 3-2: Introduction of LacY

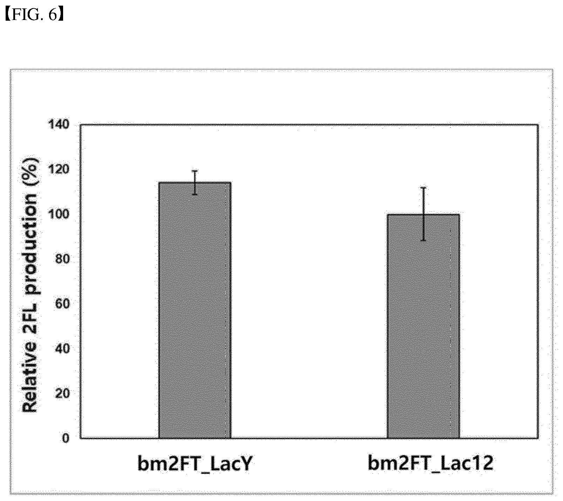

A lactose membrane transport protein derived from *Bacillus megaterium* (hereinafter, LacY) was introduced into the strain as a plasmid form, and effect thereof was analyzed after culturing at a temperature of 30°C, pH 7, and it was shown based on 100% of the activity of the mutant strain introduced by Lac12 in Example 3-1. As shown in Table 7 and FIG. 6, it was confirmed that production of 2'-fucosyllactose was increased by about 15% or more.

**[Table 7]**

| Classification | 2FL (%) |
|---|---|
| ΔLacZ_p 12455 _bm2FT _Lac 12 | 100 |
| ΔLacZ_p 12455 _bm2FT _Lac Y | 115 |

### Example 4. Selection of main GDP-fuc synthesis genes

Four kinds of GDP-fuc synthesis enzymes of the *Bacillus megaterium* 14581 strain, GDP-D-mannose-4,6-dehydratase (hereinafter, Gmd), GDP-L-fucose synthase (hereinafter, WcaG), phosphomannomutase (hereinafter, Hypo or ManB) and GTP mannose-1-phosphate guanylyltransferase (hereinafter, ManC) were transformed into the strain as a plasmid form so as to be overexpressed by using a constitutive expression promoter, for the purpose of improving the productivity. Specifically, 50 µl plasmid was mixed by 500ml *B. megaterium* protoplast treated with lysozyme. 5mL SMMP was added to 15mL Falcon containing PEG-P 1.5mL, and the Falcon was carefully rolled to mix. After the cells were collected by centrifuging at 1,300g for 10 minutes, the cells were released into the SMMP, and 250rpm shaking was performed for 1.5mL at 37°C to complete transformation.

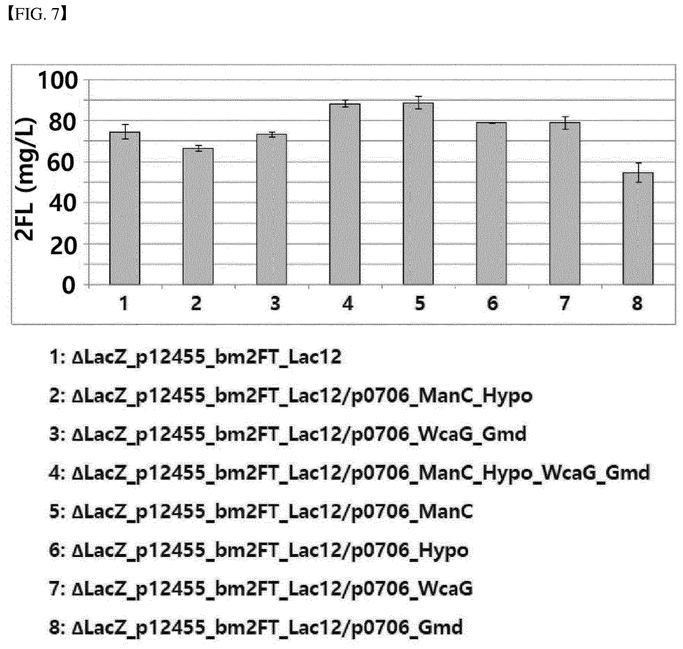

The constructed strains were reacted at a temperature of 30°C, pH 7 for 80 hours. As shown in Table 8 and FIG. 7, among various combinations which were constructed and productivity thereof was tested, when ManC, Hypo, WcaG, and Gmd were simultaneously expressed, the productivity was increased by about 15% or more compared to a control group of ΔLacZ_p12455_Bm2FT_Lac12 in which GDP-fuc synthase was not overexpressed. This tendency was shown also that in the case of expressing only ManC in a recombinant protein form, the 2'-fucosyllactose productivity was increased by about 15% or more compared to a control group of ΔLacZ_p12455_Bm2FT_Lac12 in which ManC was not introduced in a plasmid form.

**[Table 8]**

| Classification | 2FL (mg/L), 30°C, 80h |
|---|---|
| ΔLacZ_p12455_bm2FT_Lac12 | 74.6 |
| ΔLacZ_p12455_bm2FT_Lac12/p0706_ManC_Hypo | 66.5 |
| ΔLacZ_p12455_bm2FT_Lac12/p0706_WcaG_Gmd | 73.3 |
| ΔLacZ_p 12455_bm2FT_Lac_12/p0706_ManC_Hypo_WcaG_Gmd | 88.3 |
| ΔLacZ_p12455_bm2FT_Lac12/p0706_ManC | 88.7 |
| ΔLacZ_p12455_bm2FT_Lac12/p0706_Hypo | 78.9 |
| ΔLacZ_p12455_bm2FT_Lac12/p0706_WcaG | 79.0 |
| ΔLacZ_p12455_bm2FT_Lac12/p0706_Gmd | 54.8 |

### Example 5. Analysis of productivity of 2'-fucosyllactose

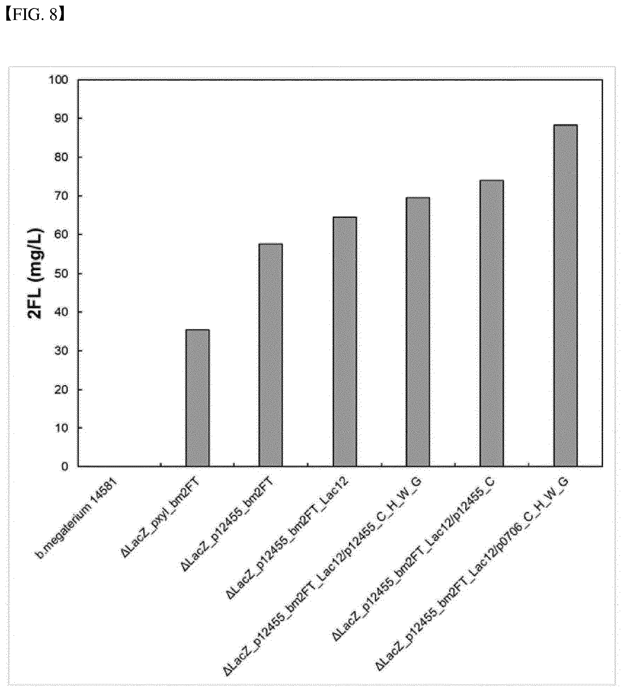

In order to compare the productivity of the finally constructed strains, after the strains were cultured for 65 hours with adding lactose a temperature of 30°C, pH 7, their production of 2'-fucosyllactose was compared. Through various combinations of the GDP-fuc enzyme and constitutive expression promoter, the productivities of 2'-fucosyllactose in the strains were compared (Table 9 and FIG. 8).

**[Table 9]**

| Classification | 2FL (mg/L), 30°C, 65h |
|---|---|
| b.megaterium 14581 | 0 |
| ΔLacZ_pxy1_bm2FT | 35.4 |
| ΔLacZ_p12455_bm2FT | 57.8 |
| ΔLacZ_p12455_bm2FT_Lac12 | 67.6 |
| ΔLacZ_p12455_bm2FT_Lac12/p12455_C_H_W_G | 70 |
| ΔLacZ_p12455_bm2FT_Lac12/p12455_C | 74 |
| ΔLacZ_p12455_bm2FT_Lac12/p0706_C_H_W_G | 88.3 |

As shown in Table 9 and FIG. 8, the wild type *B. megaterium* 14581 could not produce 2'-fucosyllactose, but could be modified to achieve significantly higher productivity of 2'-fucosyllactose compared to a conventional xylose inducible promoter, by introduction of α-1,2-fucosyl transferase, introduction of various constitutive expression promoters, deletion of LacZ, introduction of a lactose membrane transport protein, introduction of GDP-fuc synthase, and the like.

## Claims

1. A gene expression cassette expressing α-1,2-fucosyl transferase in a *Bacillus* sp. microorganism, comprising
a nucleic acid sequence encoding α-1,2-fucosyl transferase, and a regulatory sequence operably linked thereto,
wherein the regulatory sequence comprises a constitutive expression promoter.

2. The gene expression cassette according to claim 1, wherein the constitutive expression promoter is at least one selected from the group consisting of p3610, p12455, p24930, pTu, and pPDC.

3. The gene expression cassette according to claim 1, wherein the regulatory sequence comprises a ribosome binding site (RBS) comprising the nucleic acid sequence of SEQ ID NO: 21.

4. The gene expression cassette according to claim 1, wherein the nucleic acid sequence encoding α-1,2-fucosyl transferase comprises a nucleic acid sequence encoding at least one amino acid sequence selected from the group consisting of SEQ ID NOs: 1 to 5.

5. The gene expression cassette according to claim 1, wherein the nucleic acid sequence encoding α-1,2-fucosyl transferase comprises at least one nucleic acid sequence selected from the group consisting of SEQ ID NOs: 6 to 10.

6. The gene expression cassette according to claim 1, wherein the gene expression cassette further comprises a nucleic acid sequence encoding a lactose membrane transport protein.

7. The gene expression cassette according to claim 6, wherein the lactose membrane transport protein is at least one selected from the group consisting of Lac12 and LacY.

8. The gene expression cassette according to claim 6, further comprising a regulatory sequence of the lactose membrane transport protein, wherein the regulatory sequence is at least one selected from the group consisting of a promoter and RBS.

9. The gene expression cassette according to claim 1, wherein the *Bacillus* sp. microorganism is at least one selected from the group consisting of *Bacillus megaterium, Bacillus subtilis, Bacillus cereus, Bacillus licheniformis, Bacillus amyloliquefaciens,* and *Bacillus thuringiensis.*

10. A vector comprising the gene expression cassette expressing α-1,2-fucosyl transferase according to any one of claim 1 to claim 9.

11. The vector according to claim 10, wherein the vector is a recombinant vector having the cleavage map of FIG. 1a.

12. A recombinant *Bacillus* sp. microorganism, comprising the gene expression cassette according to any one of claim 1 to claim 9, or transformed with a vector comprising the expression cassette.

13. The microorganism according to claim 12, wherein the vector further comprises a fucose synthesis gene expression cassette.

14. The microorganism according to claim 12, wherein the microorganism further comprises a vector comprising a fucose synthesis gene expression cassette.

15. The microorganism according to claim 13 or claim 14, wherein the fucose synthesis gene expression cassette comprises
at least one selected from the group consisting of a nucleic acid sequence encoding GTP mannose-1-phosphate guanylyltransferase, a nucleic acid sequence encoding phosphomannomutase, a nucleic acid sequence encoding GDP-L-fucose synthase (WcaG), and a nucleic acid sequence encoding GDP-D-mannose-4,6-dehydradase, and a regulatory sequence operably linked thereto,
wherein the regulatory sequence comprises a constitutive expression promoter.

16. The microorganism according to claim 15, wherein the regulatory sequence comprised in the fucose synthesis gene expression cassette comprises at least one constitutive expression promoter selected from the group consisting of p3610, p12455, p24930, p0706, pTu, and pPDC.

17. The microorganism according to claim 14, wherein the vector comprising a fucose synthesis gene expression cassette is a recombinant vector having the cleavage map of FIG. 1b.

18. The microorganism according to claim 12, wherein the *Bacillus* sp. microorganism is at least one selected from the group consisting of *Bacillus megaterium, Bacillus subtilis, Bacillus cereus, Bacillus licheniformis*, *Bacillus amyloliquefaciens,* and *Bacillus thuringiensis.*

19. A composition for producing 2'-fucosyllactose, comprising at least one selected from the group consisting of α-1,2-fucosyl transferase obtained using the recombinant *Bacillus* sp. microorganism according to claim 12, a microbial cell of the microorganism, a culture of the microorganism, a lysate of the microorganism, and an extract of the lysate or culture.

20. A method for producing 2'-fucosyllactose, comprising a step of culturing a recombinant *Bacillus* sp. host cell which comprises the gene expression cassette according to any one of claim 1 to claim 9, or is transformed with a vector comprising the expression cassette.
